# EUROPÄISCHE PATENTSCHRIFT

(11) **EP 0 503 411 B1**
(45) Veröffentlichungstag und Bekanntmachung des Hinweises auf die Patenterteilung: **29.11.1995**
(21) Anmeldenummer: 92103514.3
(22) Anmeldetag: 29.02.1992
(51) Int. Cl.: C07D 211/56, C07D 211/58, C07D 401/04, C07D 401/06, A61K 31/445

(54) **Substituierte N-Phenylpiperidine und Arzneimittel daraus**
Substituted N-phenylpiperidines and medicaments thereof
N-Phénylpipéridines substituées et médicaments à partir de celles-ci

(30) Priorität: 31.05.1991 DE 4117904; 14.03.1991 DE 4108184
(43) Veröffentlichungstag der Anmeldung: 16.09.1992
(73) Patentinhaber: BASF Aktiengesellschaft, 67063 Ludwigshafen (DE)
(72) Erfinder: Lubisch, Wilfried, Dr., W-6800 Mannheim 1 (DE); Schult, Sabine, Dr., W-6900 Heidelberg (DE); Behl, Berthold, Dr., W-6700 Ludwigshafen (DE); Kirchengast, Michael, Dr., W-6800 Mannheim 1 (DE)

(56) Entgegenhaltungen:
- EP-A- 481 299
- EP-A- 0 097 000
- AT-B- 170 868
- BE-A- 678 063
- DE-C- 749 887
- FR-M- 6 553
- US-A- 4 902 800
- BERICHTE DER DEUTSCHEN CHEMISCHEN GESELLSCHAFT, Bd. 74, Nr. 6, 1941, BERLIN, DE Seiten 1658 - 1660; V. HAHN ET AL.: 'Über eine neue Reihe von ...'
- HELVETICA CHIMICA ACTA, Bd. XXVI, Nr. IV, 1943, Seiten 1132 - 1141; V. HAHN ET AL.: 'Über substituierte ...'

## Beschreibung

Die vorliegende Erfindung betrifft N-Phenylpiperidine der allgemeinen Formel I 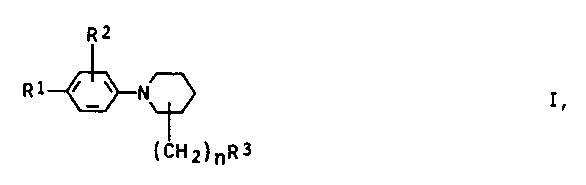
in der die Variablen die folgende Bedeutung haben:
- R¹: Wasserstoff, Nitro, Cyano, Halogen, C₁-C₄-Alkyl, Trifluormethyl, Trifluormethoxy, Hydroxy, Hydroxymethyl, Hydroxycarbonyl, Formyl, Formylamino, Amino, Aminocarbonyl, Tetrazin-5-yl, R⁴-O-, R⁴-O-CH₂-, R⁴-O-CO-, R⁴-CO-, R⁴-NH-CO, R⁴-CO-NH-, R⁴-SO₂-NH-;
- R²: Wasserstoff, Nitro, Halogen, C₁-C₄-Alkyl oder R⁴-O-;
- R³: einen der folgenden Reste: 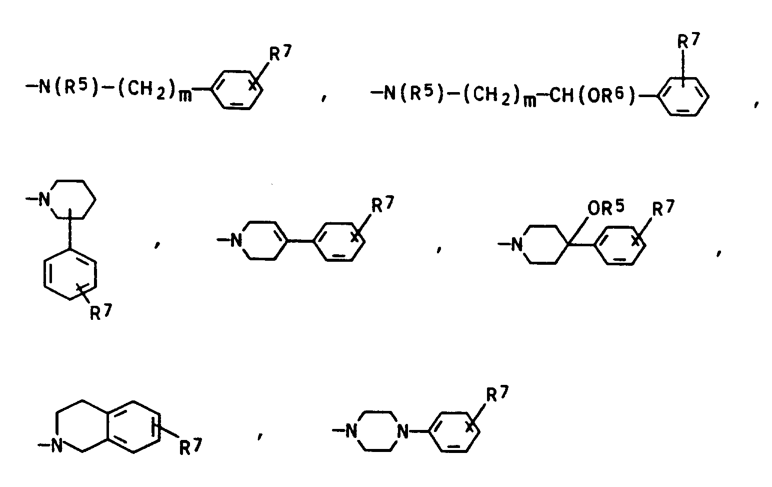
- R⁴: C₁-C₄-Alkyl oder Phenyl, das noch einen der Reste R² tragen kann;
- R⁵,R⁶: Wasserstoff, C₁-C₄-Alkyl oder Phenyl, das noch einen der Reste R² tragen kann;
- R⁷: einer der Reste R¹
- n: 0 oder 1;
- m: 1 oder 2;
mit der Maßgabe, daß R³ nur dann 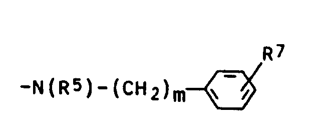
bedeutet, wenn n 1 ist,
im Falle optischer Isomerie auch die optischen Isomeren, sowie die physiologisch verträglichen Säureadditionssalze.

Weiterhin betrifft die Erfindung die Verbindungen I zur Verwendung auf dem Arzneimittelgebiet, die Verbindungen I enthaltende Arzneimittel sowie die Verwendung der Verbindungen I und deren Salzen mit physiologisch verträglichen Säuren zur Herstellung von Arzneimitteln, vor allem zur Behandlung von Herzrhythmusstörungen.

Arzneimittel zur Behandlung von Herzrhythmusstörungen (Antiarrhythmika) teilt man aufgrund ihrer Wirkungsweise nach Vaughan-Williams [vgl. J. Clin. Pharmacol. 24, 129 (1984) und ED. E.M. Vaughan-Williams-Handbook of Exp. Pharmacol. 89, Kapitel 2 (1989)] in vier Klassen ein:
(I) Natrium-Antagonisten,
(II) Adrenerge beta-Rezeptorblocker,
(III) Repolarisationshemmstoffe und
(IV) Calcium-Antagonisten.

Die bisher verwendeten Antiarrhythmika, die meistens der Klasse I angehören, sind nur in einem engen therapeutischen Dosierungsbereich anwendbar.

Wünschenswert sind Antiarrhythmika der Klasse III, da diese üblicherweise weniger Nebenwirkungen aufweisen als Antiarrhythmika der Klasse I und auch bei Herzrhythmusstörungen, insbesondere bei sogenannten "reentry"-Arrhythmien (wiederkehrende Kammertachykardie und Kammerflimmern), wirken, die mit Vertretern der anderen Klassen nicht zufriedenstellend behandelt werden können. Beispiele für Wirkstoffe der Klasse III sind Amiodaron [(2-Butyl-3-benzofuranyl)-(4-(2-diethylaminoethoxy)-3,5-diiodophenyl)-keton; vgl. Circulation 68, 88 (1983)1 und D-Sotalol [4'-(1-Hydroxy-2-(isopropylamino)-ethyl)methansulfonanilid; vgl. Am. Heart. J. 109, 949 (1985) und J. Clin. Pharmacol. 27, 708 (1987)].

Amino-substituierte N-Phenylpiperidine vom Typ der Verbindungen I sind aus folgenden Druckschriften bekannt:
BE-A 678 063 (Protozoenwachstumshemmer),
EP-A 97 000 (antiarrhythmische Wirkung),
US 4,902,800 (Interleukin-I-Inhibitoren).

Weitere N-Phenylpiperidine vom Typ der Verbindungen I, die jedoch eine antihistaminische Wirkung aufweisen, sind aus DRP 749 887 (1941), Chem. Ber. 74, 1648, 1658 und 1661 (1941) und Helv. Chim. Acta 26, 1132 (1943) bekannt.

In der nachveröffentlichten EP 481 299 werden N-Phenyl-piperidinyl-4-amine als Antiarrhythmika beschrieben.

Der Erfindung lag die Aufgabe zugrunde, neue und hochwirksame Antiarrhythmika, die als Repolarisationshemmstoffe wirken, bereitzustellen.

Demgemäß wurden die eingangs definierten substituierten N-Phenylpiperidine gefunden.

Außerdem wurde die Verwendung der Verbindungen I auf dem Arzneimittelgebiet, die Verbindungen I enthaltende Arzneimittel sowie die Verwendung der Verbindungen I zur Herstellung von Arzneimitteln gefunden.

Neben ihrer Wirkung als Antiarrhythmika hat man bei den substituierten N-Phenylpiperidinen I eine Affinität zum Sigma-Rezeptor gefunden, weshalb sie auch als Antipsychotika, Antikonvulsiva, Anxiolytika und Neuroprotektiva dienen können.

Substanzen, die an Sigma-Rezeptoren binden (z.B. Cyclazocine, Pentazocin, Ketamin) zeigen psychotomimetische Effekte beim Menschen [J. Pharmacol. Exp. Ther. 197, 517 (1976)]. Andererseits haben Antipsychotika wie Haloperidol und BMY 14802 [α-(4-Fluorphenyl)-4-(5-fluoro-2-pyrimidinyl)1-piperazin-butanol] eine hohe Affinität für Sigma-Bindungsplätze [J. Pharmacol. Exp. Ther. 238, 739 (1986)]. Außerdem weist die relativ hohe Präsenz von Sigma-Rezeptoren in der "Substantia Nigra Compacta" auf eine mögliche Verbindung dieser Rezeptoren mit dem dopaminergen Neurotransmittersystem hin [J. Neurosci. 9, 326 (1989)]. Das Vorkommen der Sigma-Bindungsplätze in anderen Bereichen des Gehirns und die hohe Dichte in anderen Organen wie der Milz, Nierenrinde, Leber und Lymphzyten [Endocrinology 124, 1160 (1989) und Pharmacol. 23, 619, (1983)] läßt aber auch auf eine breite physiologische Bedeutung des Sigma-Rezeptors schließen. Bei einer Vielzahl pathophysiologischer Prozesse kann deshalb mit einer therapeutischen Anwendbarkeit von Sigma-Liganden gerechnet werden.

Außer als Antipsychotika kommen die von uns entwickelten Sigma-Liganden als Antikonvulsiva (krampflösende Stoffe), Anxiolytika (angstlösende Stoffe) und Cytoprotektiva bei Ischämien in Betracht.

Im Hinblick auf die bestimmungsgemäße Verwendung der substituierten N-Phenylpiperidine I als Antiarrhythmica, Antipsychotika, Antikonvulsiva, Anxiolytika und Neuroprotektiva kommen als Substituenten vorzugsweise die folgenden Reste in Betracht:
- R¹: Wasserstoff, Nitro, Cyano;
Halogen wie Fluor, Chlor, Brom und Jod, insbesondere Fluor, Chlor und Brom;
verzweigtes oder unverzweigtes C₁-C₄-Alkyl wie Methyl, Ethyl, n-Propyl, 1-Methylethyl, n-Butyl, 1-Methylpropyl, 2-Methylpropyl und 1,1-Dimethylethyl, insbesondere Methyl und Ethyl;
Trifluormethyl, Trifluormethoxy, Hydroxy, Hydroxymethyl, Hydroxycarbonyl, Formyl, Formylamino, Amino, Aminocarbonyl, Tetrazin-5-yl; R⁴-O-, R⁴-O-CH₂-, R⁴-O-CO-, R⁴-CO-, R⁴-NH-CO, R⁴-CO-NH-, R⁴-SO₂-NH-; bevorzugte Reste R¹ sind Nitro, Cyano, Halogen, Amino und R⁴-SO₂-NH-;
- R²: Wasserstoff, Nitro oder R⁴-O-;
Halogen wie vorstehend genannt, insbesondere Fluor und Chlor; verzweigtes oder unverzweigtes C₁-C₄-Alkyl wie vorstehend genannt, insbesondere Methyl und Ethyl;
besonders bevorzugt ist Wasserstoff;
- R³: einen der folgenden Reste: 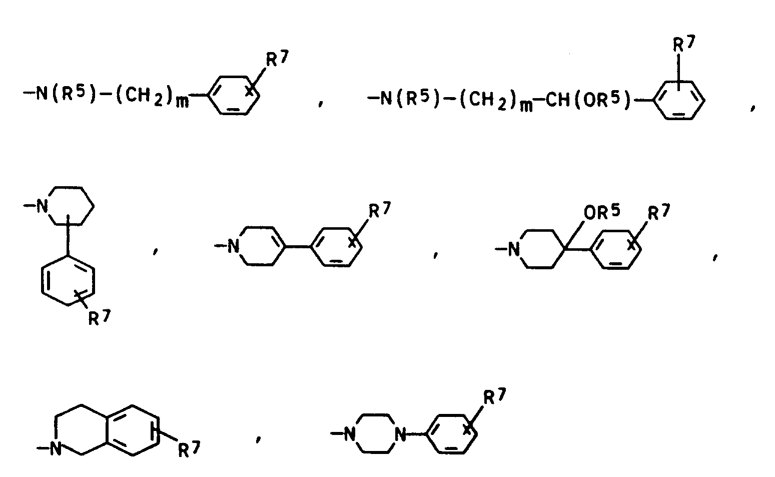
- R⁴: verzweigtes oder unverzweigtes C₁-C₄-Alkyl wie vorstehend genannt, insbesondere Methyl und Ethyl;
Phenyl, das noch einen der Reste R² tragen kann;
- R⁵,R⁶: Wasserstoff oder einer der Reste R⁴;
- R⁷: einer der Reste R¹.

Die substituierten N-Phenylpiperidine I sind auf verschiedene Weise erhältlich, und zwar bevorzugt nach einem der folgenden Verfahren: 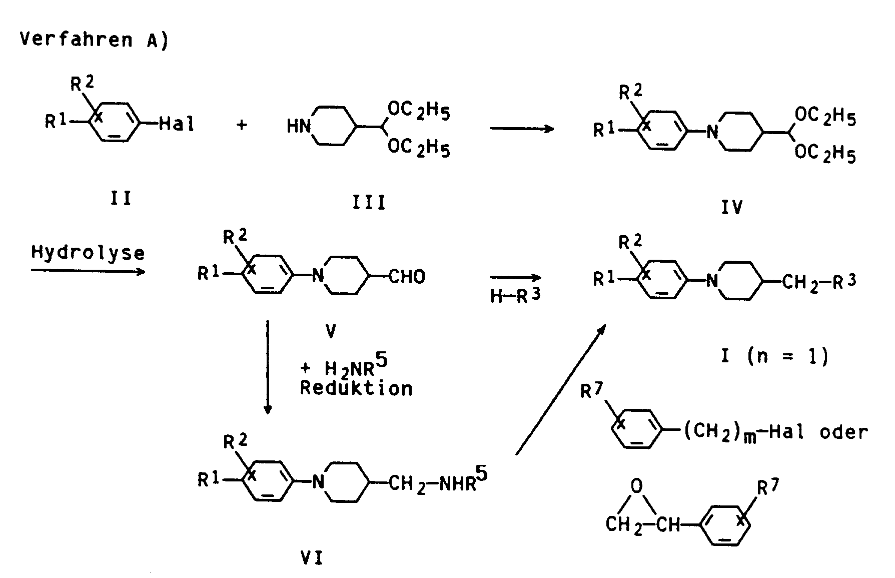
Hal = Fluor, Chlor oder Brom
In der ersten Stufe setzt man ein Arylhalogenid II mit einem Piperidin III zu einem Phenylpiperidin IV um.

Bei Arylhalogeniden II, die einen elektronenziehenden Substituenten R¹, beispielsweise Nitro, Cyano, Formyl, Hydroxycarbonyl oder R⁴-O-CO-, tragen, empfiehlt sich die Reaktionsführung in einem polaren Lösungsmittel wie Dimethylformamid, niederen Alkoholen, z.B. Methanol oder Ethanol, oder Ketonen, z.B. Aceton. Besonders bevorzugt arbeitet man hierbei in Gegenwart einer Base wie Kaliumcarbonat. Im allgemeinen liegt die Reaktionstemperatur zwischen 60 und 150°C.

Bei Arylhalogeniden II ohne elektronenziehenden Substituenten R¹, empfiehlt sich die Reaktionsführung in Abwesenheit eines Lösungsmittels oder in einem Lösungsmittel wie Wasser oder einem Glykol.

Im allgemeinen arbeitet man hierbei in Gegenwart einer katalytischen Menge eines Metalls oder Metallsalzes, wobei Kupfer als Metallkomponente besonders bevorzugt ist. Die Reaktionstemperatur liegt normalerweise zwischen 100°C und der Siedetemperatur des jeweiligen Lösungsmittels.

Das erhaltene Phenylpiperidin IV wird anschließend zweckmäßig durch saure Hydrolyse, im allgemeinen durch Verwendung wäßriger Säuren, vorzugsweise wäßrige Salz- oder Schwefelsäure, in den Aldehyd V übergeführt. Durch reduktive Aminierung mit Aminen R³H erhält man anschließend die erfindungsgemäßen Verbindungen I. Die reduktive Aminierung wird im allgemeinen bei Temperaturen von 5 bis 80°C, vorzugsweise 10 bis 30°C, in Gegenwart von Reduktionsmitteln wie Natriumcyanoborhydrid oder Wasserstoff in Gegenwart von Hydrierkatalysatoren wie Pd/Kohle, Pt/Kohle oder Raney-Nickel, zweckmäßig in polaren organischen Lösungsmitteln wie Alkohole oder Dimethylformamid durchgeführt.

Alternativ können die Verbindungen I in der Weise erhalten werden, daß der Aldehyd V mit einem Amin H₂NR⁵ durch reduktive Aminierung, zweckmäßig entsprechend den vorstehend beschriebenen Bedingungen, zum Amin VI umgesetzt wird, das anschließend durch Alkylierung mit einem Halogenid 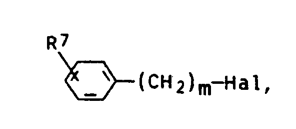
vorzugsweise als Chlorid oder Bromid oder einem Epoxid 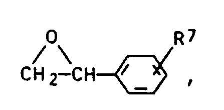
in die erfindungsgemäßen Verbindungen I überführt wird.

Zweckmäßig arbeitet man hierbei in polaren, organischen Lösungsmitteln, beispielsweise in einem Alkohol oder in Dimethylformamid, gegebenenfalls unter Zusatz von basischen Stoffen, z.B. Erdalkalimetallhydroxiden oder -carbonaten wie NaOH, KOH, Na₂CO₃ und K₂CO₃. Im allgemeinen liegt die Reaktionstemperatur zwischen 20 und 150°C.

Die Reaktionsführung erfolgt vorteilhaft bei Atmosphärendruck oder unter dem Eigendruck des jeweiligen Lösungsmittels; höherer oder niedrigerer Druck ist auch möglich, bringt normalerweise aber keine Vorteile. 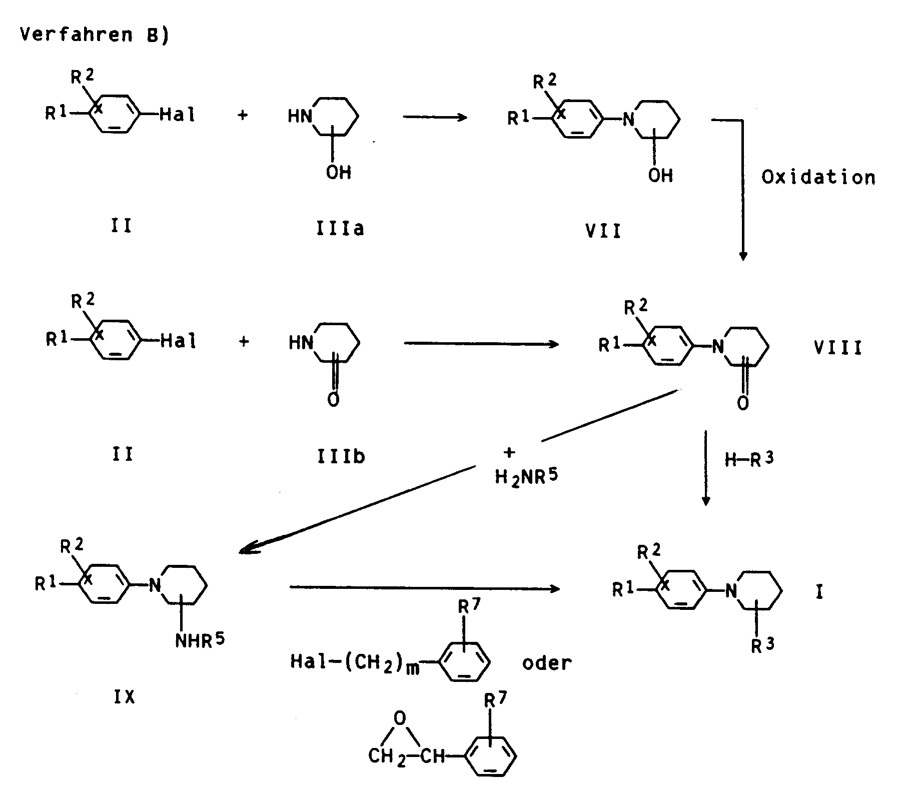
Gemäß Verfahren B) wird das Halogenid II analog zu den Reaktionsbedingungen gemäß Verfahren A) entweder mit dem Piperidinol IIIa zunächst zu VII umgesetzt, das anschließend zu VIII oxidiert wird, oder mit dem Piperidon IIIb direkt zu VIII umgesetzt. Die Oxidation des Alkohols VII zum Keton VIII erfolgt bevorzugt nach Pfitzner-Moffat oder Swern oder entsprechenden Verfahren, die in T.T. Tidwell, Synthesis 1990, 857 beschrieben sind.

Die Umsetzung des Piperidons VIII zu den erfindungsgemäßen Verbindungen I erfolgt analog zu den im Verfahren A) beschriebenen Methoden durch reduktive Aminierung entweder mit dem Amin R³-H oder H₂NR⁵, wobei im letzteren Fall wiederum das erhaltene Amin noch mit dem Halogenid 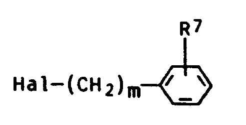
oder dem Epoxid 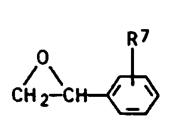
alkyliert wird.

Bezüglich des Druckes gelten die Angaben für Verfahren A). 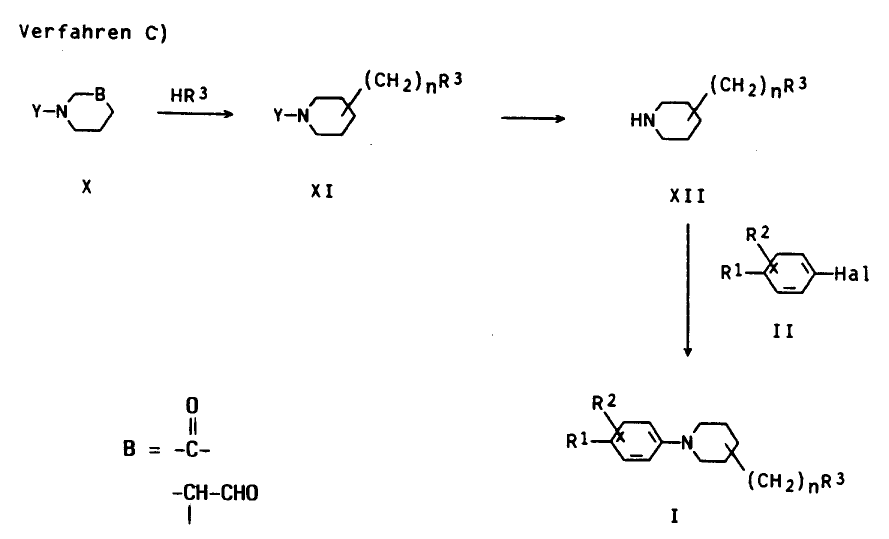
In einer weiteren Variante erhält man gemäß Verfahren C) die Verbindungen I durch Umsetzung des Piperidins XII mit dem Halogenid II unter analogen Reaktionsbedingungen wie gemäß Verfahren A). Ausgehend vom Piperidin X, bei dem Y eine Schutzgruppe wie Benzyl, CH₃-CO-, CF₃-CO- oder tert.-Butoxy-Carbonyl- bedeutet, entsteht durch reduktive Aminierung mit dem Amin HR³ unter analogen Bedingungen wie beim Verfahren A) das Piperidin XI, das durch Abspaltung der Schutzgruppe Y in das Piperidin XII überführt wird. Die Abspaltung der Schutzgruppe Y erfolgt im allgemeinen entweder katalytisch mit Wasserstoff in Gegenwart von Katalysatoren wie Pd/Kohle, Pt/Kohle oder Raney-Nickel oder durch saure oder basische Hydrolyse, z.B. mit wäßrigen Säuren wie Salzsäure, Schwefelsäure oder mit basischen Mitteln, z.B. Erdalkalimetallhydroxiden oder -carbonaten wie NaOH, KOH, Na₂CO₃ und K₂CO₃. Zweckmäßig arbeitet man bei erhöhten Temperaturen, z.B. zwischen 25 bis 100°C. Bezüglich des Druckes gelten die Angaben für Verfahren A). 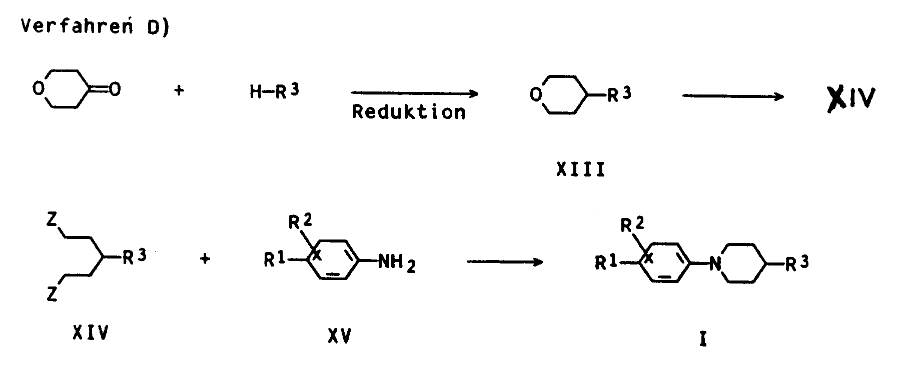
(Z = Halogen, insbesondere Chlor und Brom)
Ein weiterer Darstellungsweg (Verfahren D)) geht vom Tetrahydro-4-pyranon aus, das in üblicher Weise, z.B. analog zur Darstellung von Verbindung I aus VIII in Verfahren B), durch reduktive Aminierung zu XIII umgesetzt wird. XIII wird zweckmäßig in einer konzentrierten Säure wie Bromwasserstoffsäure oder Salzsäure, gegebenenfalls in einem inerten Lösungsmittel wie einem Alkohol, bei erhöhter Temperatur, z.B. bei Temperaturen von 50 bis 100°C, in das Dihalogenid XIV überführt.

Die nachfolgende Alkylierung des Anilins XV mit XIV zu den erfindungsgemäßen Verbindungen I erfolgt zweckmäßig in polaren Lösungsmitteln wie Alkoholen und Dimethylformamid oder ohne Lösungsmittel, gegebenenfalls in Gegenwart einer Base wie NaOH und Kaliumcarbonat. Hierbei liegt die Reaktionstemperatur im allgemeinen zwischen 50 und 150°C.

Bezüglich des Druckes gelten die Angaben für Verfahren A). 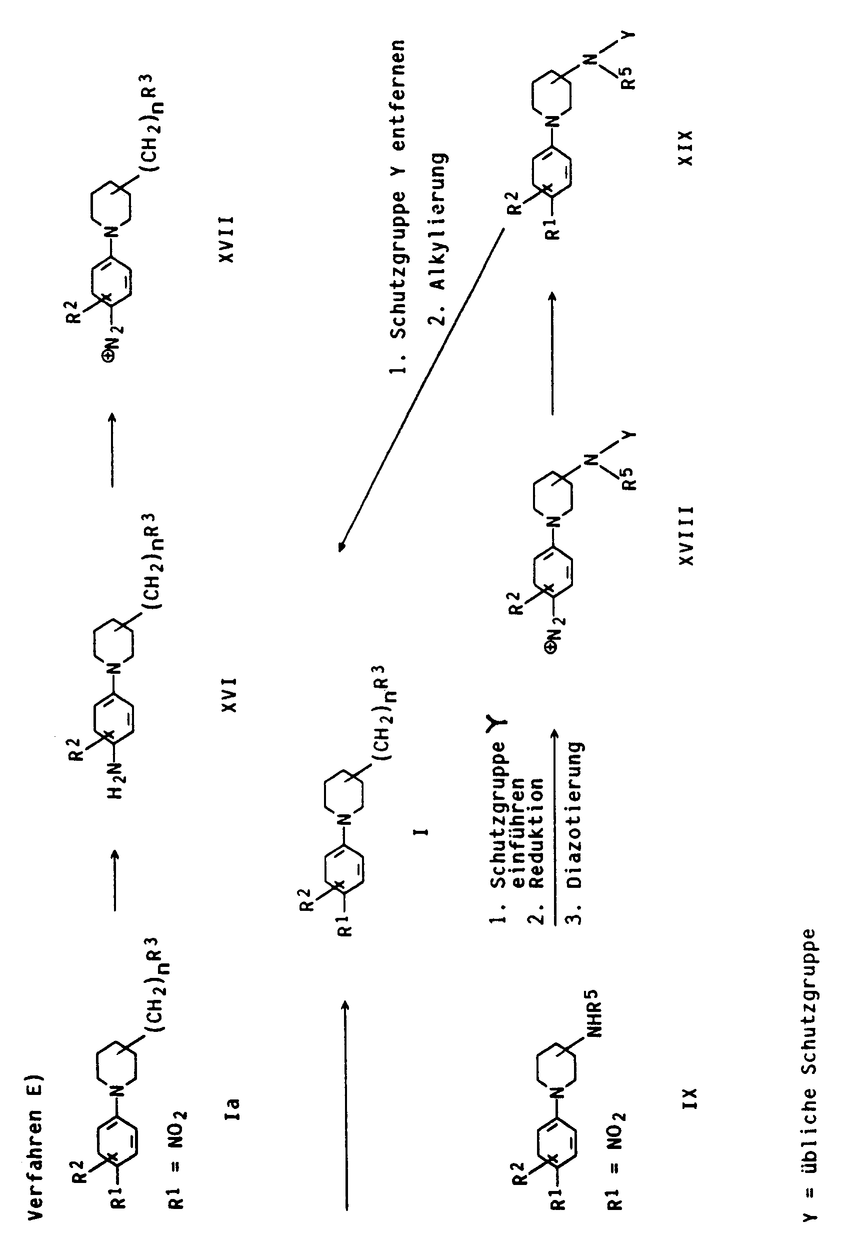
Gemäß Verfahren E) werden ausgehend von den Nitroverbindungen Ia (R¹ = NO₂) und IX (R¹ = NO₂) synthetische Wege dargestellt, die erlauben, ausgehend von der Nitro-Gruppe andere Substituenten für R¹ einzuführen.

In einer ersten Alternative wird das Derivat Ia (R¹ = NO₂) zum Anilin XVI reduziert, wobei man entweder katalytisch mit Wasserstoff an Hydrierkatalysatoren wie Pd/Kohle oder Pt/Kohle in bevorzugt polaren Lösungsmitteln wie Alkoholen umsetzt oder die Reduktion chemisch mit Natriumborhydrid/Kupferkatalyse (Sung, Yoo et al., Synlett 1990, 419) oder mit Reagenzien, die z.B. im Houben-Weyl, Methoden der organischen Chemie, Bd. 11/1, Kap. IV aufgelistet sind (wie Sn/HCl, Fe/HCl und Na₂S₂O₄), durchgeführt. Das Anilin XVI wird in üblicher Weise (wie in Houben-Weyl, Methoden der organischen Chemie, Bd. 10/3, Kap. 1/A beschrieben) diazotiert und anschließend in konventionaller Weise (Houben-Weyl, Bd. 10/3, Kap. 1/B und dort zitierte Literatur) in andere von Ia verschiedene erfindungsgemäße Verbindungen I überführt, in denen R¹ z.B. H, Cl und F bedeutet.

In einer weiteren Alternative des Verfahrens E) wird Verbindung I ausgehend von Phenylpiperidin IX (R¹ = NO₂) hergestellt. Die Umwandlung des Phenylpiperidins IX zu I erfolgt in mehreren Schritten:
1. Einführung einer Schutzgruppe Y für die sekundäre oder primäre Aminogruppe in IX, wobei Y eine übliche Schutzgruppe wie CH₃-CO-, CF₃-CO- oder tert.-Butoxycarbonyl darstellt und in konventioneller Weise, z.B. analog Verfahren C), eingeführt wird,
2. Reduktion der Nitro-Gruppe,
3. Diazotierung des resultierenden Anilins und
4. Überführung des Diazoniumsalzes XVIII in XIX.

Die Stufen 2 bis 4 erfolgen in analoger Weise wie in der ersten Alternative von Verfahren E) beschrieben. Die Schutzgruppe Y in XIX wird auf üblichen Wegen, z.B. in analoger Weise wie beim Verfahren C) entfernt und das so erhaltene Amin mit einem Rest wie 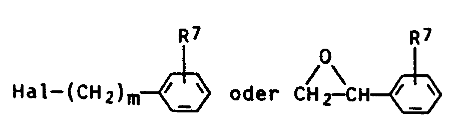
analog Verfahren B) (Überführung von IX in I) alkyliert.

Bezüglich des Druckens gelten die Angaben für Verfahren A).

Die Darstellung der optischen Isomere erfolgt in üblicher Weise durch Salzbildung des racemischen Amins mit optisch aktiven Säuren wie z.B. Weinsäure, Dibenzoylweinsäure, Mandelsäure, Ditolylweinsäure oder Camphersulfonsäure und anschließende Trennung über Umkristallisation (siehe P. Newman, Optical Resolution Procedures for Chemical Compounds, Vol. 1, New York, ca. 1979). Die Auftrennung kann entweder bei der Endstufe, also den beanspruchten Aminen I, oder bei einer Vorstufe der Verfahren A bis E, z.B. VI, IX und HR³, erfolgen, wobei die folgenden Umsetzungen zum Endprodukt I in analoger Weise zu den Racematen mit allerdings nur einem Antipoden ausgeführt werden. Die Charakterisierung der Enantiomeren wird üblicherweise entweder über die Messung des spezifischen Drehwerts oder durch Bestimmung des Enantiomerenverhältnisses durch HPLC an chiralen Trägern bzw. NMR mit optisch aktiven Shift-Reagenzien vorgenommen.

Zur Darstellung von physiologisch verträglichen Säureadditionssalzen kann man die substituierten N-Phenylpiperidine I mit üblicherweise verwendeten Salzbildnern wie Chlorwasserstoffsäure, Bromwasserstoffsäure, Schwefelsäure, Phosphorsäure, Essigsäure, Malonsäure, Bernsteinsäure, Fumarsäure, Maleinsäure, Zitronensäure, Weinsäure, Milchsäure und Oxalsäure in an sich bekannter Weise umsetzen.

Die Verbindungen I kann man in freier Form oder bevorzugt in Form eines Salzes mit einer physiologisch verträglichen Säure (s.o.) peroral, parenteral oder intravenös verabreichen.

Die Dosierung hängt vom Alter, Zustand und Gewicht des Patienten sowie von der Applikationsform ab. In der Regel beträgt die tägliche Wirkstoffdosis zwischen 0,01 und 25, vorzugsweise zwischen 0,1 und 20, insbesondere zwischen 1 und 10 mg/kg Körpergewicht bei oraler, und zwischen 0,5 und 5, vorzugsweise zwischen 1 und 3 mg/kg Körpergewicht bei intravenöser Anwendung.

Die Verbindungen I können in den gebräuchlichen galenischen Applikationsformen fest oder flüssig angewendet werden, z.B. als Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Emulsionen und Sprays.

Diese werden in üblicher Weise hergestellt. Die Wirkstoffe können dabei mit den gebräuchlichen galenischen Hilfsmitteln wie Tablettenbindern, Füllstoffen, Weichmachern, Netzmitteln, Dispergiermitteln, Emulgatoren, Lösungsmitteln, Retardierungsmitteln und/oder Antioxidantien verarbeitet werden (vgl. H. Sucker et al., Pharmazeutische Technologie, Thieme Verlag Stuttart 1978). Die so erhaltenen Zubereitungen enthalten den Wirkstoff normalerweise in einer Menge von 0,1 bis 99 Gew.-%.

Die Verbindungen I sind Antiarrhythmika der Klasse III (Klasse der Repolarisationshemmstoffe). Sie zeigen außerdem eine Affinität zum Sigma-Rezeptor und weisen daher eine antipsychotische, antikonvulsive, anxiolytische und neuroprotektive Wirkung auf.

### Beispiele

### A) Synthesebeispiele

### Beispiel 1

### 1-(4-Nitrophenyl)-4-((N-methyl-N-4-nitrobenzylamino)methyl)piperidin

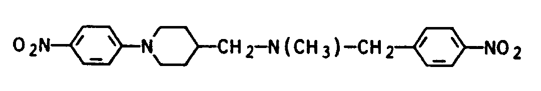

Eine Lösung aus 5,0 g (21,3 mol) 4-Formyl-1-(4-nitrophenyl)-piperidin, 3,5 g (21,3 mol) N,N-(4-Nitrobenzyl)methylamin, 1,3 g Eisessig und 150 ml Methanol wurde bei etwa 20°C portionsweise mit 1,3 g Natriumcyanoborhydrid versetzt. Nach 16 Stunden Rühren entfernte man das Lösungsmittel unter reduziertem Druck und verteilte den Rückstand zwischen Wasser und Essigsäureethylester. Die organische Phase wurde wie üblich auf das Produkt hin aufgearbeitet.
Ausbeute: 7,3 g; Fp.: 162-163°C

### Vorstufe 1.1

### 4-(Diethoxymethyl)-1-(4-nitrophenyl)piperidin

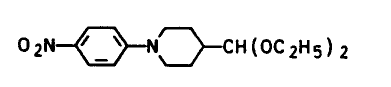

Eine Mischung aus 50,0 g (0,26 mol) 4-(Diethoxymethyl)-piperidin, 37,3 g (0,26 mol) 1-Fluor-4-nitrobenzol, 37,0 g (0,52 mol) Kaliumkarbonat und 500 ml Dimethylformamid wurde 4 Stunden auf 100°C erhitzt. Anschließend entfernte man das Lösungsmittel unter reduziertem Druck und verteilte den Rückstand zwischen Wasser und Essigssäureethylester. Danach wurde die organische Phase abgetrennt, getrocknet und eingeengt.
Ausbeute: 79,2 g; Öl.

| | | | | |
|---|---|---|---|---|
| Analyse: | berechnet | 62,3% C, | 7,9% H, | 9,1% N; |
| | gefunden | 62,3% C, | 8,1% H, | 9,6% N. |

### Vorstufe 1.2

### 4-Formyl-1-(4-nitrophenyl)-piperidin

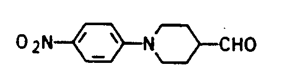

80,0 g (0,26 mol) 4-(Diethoxymethyl)-1-(4-nitrophenyl)piperidin wurden in einem Gemisch aus 500 ml Wasser und 100 ml konzentrierter wässriger Salzsäure 30 Minuten auf Rückflußtemperatur erhitzt. Nachdem die Lösung mit Natronlauge alkalisch gemacht wurde, extrahierte man das Produkt mit Diethylether. Anschließend wurde das Produkt wie üblich isoliert. Ausbeute: 90 %; Fp.: 72-73°C

### Beispiel 2

### 4-(N-Benzyl-N-methylaminomethyl)-1-(4-nitrophenyl)-piperidin

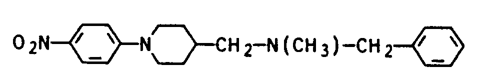

4,0 g (17,1 mmol) 4-Formyl-1-(4-nitrophenyl)-piperidin, 2,1 g (17,1 mmol) N,N-Benzylmethylamin, 1,0 g (17,1 mmol) Essigsäure und 1,1 g (17,1 mmol) Natriumcyanoborhydrid wurden analog Beispiel 1 umgesetzt.
Ausbeute: 69 %; Fp.: 91-92°C

### Beispiel 3

### 1-[1-(4-Cyanophenyl)-4-piperidinyl]-4-(4-nitrophenyl)-piperazin

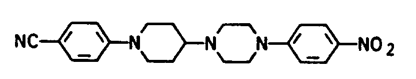

Analog Beispiel 1 wurden 2,5 g 1-(4-Cyanophenyl)-4-piperidon [vgl. Taylor et al., Synthesis, 606, (1981)] und 5,2 g 1-(4-Nitrophenyl)piperazin umgesetzt.
Ausbeute 58 %; Fp.: 243°C

### Beispiel 4

### 4-(4-Fluorphenyl)-1-[1-(4-nitrophenyl)-4-piperidinyl]-piperazin

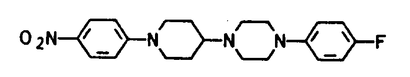

Analog Beispiel 1 wurden 3,0 g (13,6 mmol) 1-(4-Nitrophenyl)-4-piperidon [vgl. Taylor et al., Synthesis, 606, (1981)], 2,5 g (13,6 mmol) N-(4-Fluorphenyl)-piperazin, 0,8 g (13,6 mmol) Essigsäure und 0,9 g (13,6 mmol) Natriumcyanoborhydrid umgesetzt.
Ausbeute: 62 %; Fp.: 191-192°C

### Beispiel 5

### 4-(4-Chlorphenyl)-1-[1-(4-nitrophenyl)-4-piperidinyl]-piperidin-4-ol

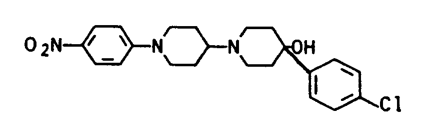

Analog Beispiel 1 wurden 3,0 g (13,6 mmol) 1-(4-Nitrophenyl)-4-piperidon [vgl. Taylor et al., Synthesis, 606, (1981)], 2,9 g (13,6 mmol) 4-(4-Chlorphenyl)-4-hydroxy-piperidin, 0,8 g (13,6 mmol) Essigsäure und 0,9 g (13,6 mmol) Natriumcyanoborhydrid umgesetzt.
Ausbeute: 18 %; Fp.: 225-226°C

### Beispiel 6

### 2-[1-(4-Nitrophenyl)-4-piperidinyl]-1,2,3,4-tetrahydroisochinolin

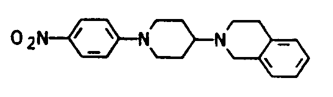

Analog Beispiel 1 wurden 3,0 g 1-(4-Nitrophenyl)-4-piperidon und 1,8 g 1,2,3,4-Tetrahydroisochinolin umgesetzt.
Ausbeute: 74 %; Fp.: 162-163°C

### Beispiel 7

### 4-(4-Fluorphenyl)-1-[1-(4-nitrophenyl)-4-piperidinyl]-1,2,5,6-tetrahydropyridin

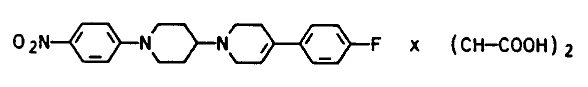

Analog Beispiel 1 wurden 5,5 g 1-(4-Nitrophenyl)-4-piperidon und 4,4 g 4-(4-Fluorphenyl)-1,2,5,6-tetrahydropyridin umgesetzt. Das Produkt kristallisierte als Fumarat.
Ausbeute: 6,3 g; Fp.: 182-183°C

### Beispiel 8

### 1-(4-Nitrophenyl)-4-(4-phenyl-1-piperidinyl)-piperidin

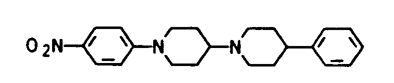

Analog Beispiel 1 wurden 3,0 g 1-(4-Nitrophenyl)-4-piperidon und 2,2 g 4-Phenylpiperidin umgesetzt. Das Produkt enthielt 1/6 mol Kristallwasser.
Ausbeute: 1,7 g; Fp.: 210-211°C

### Beispiel 9

### 1-(4-Aminophenyl)-4-(4-phenyl-1-piperidinyl)-piperidin

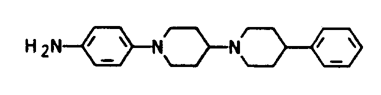

Eine Lösung aus 1,2 g (2,7 mmol 1-(4-Nitrophenyl)-4-(4-phenyl-1-piperidinyl)-piperidin (vgl. Bsp. 8) in 100 ml Methanol wurde mit 0,5 g Palladium/Kohle (enthaltend 10 Gew.-% Palladium) versetzt. Man hydrierte bis zur Beendigung der Wasserstoffaufnahme, filtrierte dann die festen Bestandteile ab und entfernte das Lösungsmittel unter reduziertem Druck.
Ausbeute: 92 %; Fp.: 156-157°C

### Beispiel 10

### 1-(4-Methansulfonamidophenyl)-4-(N-(4-methansulfonamidobenzyl)-N-methylaminomethyl)-piperidin

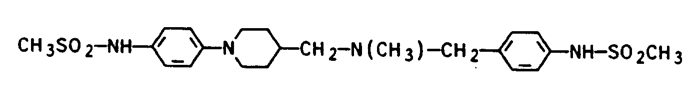

Eine Suspension von 5,4 g (14,0 mmol) der Base von 1-(4-Nitrophenyl)-4-(N,N-methyl-(4-nitrobenzyl)aminomethyl)piperidinfumarat in 150 ml Ethanol wurde bei 20 bis 25°C mit einer Lösung von 0,8 g (3,2 mmol) Kupfersulfatpentahydrat in 2 ml Wasser und danach mit 5,3 g (140 mmol) Natriumcyanoborhydrid versetzt. Man erhitzte die Mischung 3 Stunden auf Rückflußtemperatur, trennte dann gebildeten Feststoff ab und entfernte das Lösungsmittel unter reduziertem Druck. Der Rückstand wurde zwischen Wasser und Essigsäureethylester verteilt, wonach die organische Phase abgetrennt und unter reduziertem Druck eingeengt wurde.

Eine Lösung von 4,0 g (12,3 mmol) des so hergestellten 1-(4-Aminophenyl)-4-(N,N-methyl-(4-aminobenzyl)aminomethyl)piperidins und 10,0 g (98,6 mmol) Triethylamin in 350 ml wasserfreiem Tetrahydrofuran wurde bei 0°C tropfenweise mit einer Lösung von 2,8 g (12,3 mmol) Methansulfonsäurechlorid in 30 ml wasserfreiem Tetrahydrofuran versetzt. Man rührte die Mischung noch 2 Stunden bei 0°C und verdünnte sie dann mit 200 ml gesättigter wäßriger Natriumhydrogenkarbonatlösung. Nach Entfernen des Lösungsmittels unter reduziertem Druck extrahierte man den Rückstand mit Methylenchlorid. Die organische Phase wurde getrocknet und eingeengt. Die Reinigung des Rohproduktes erfolgte durch Chromatographie an Kieselgel (Laufmittel: Methylenchlorid/Methanol 10:1).
Ausbeute: 1,6 g;

| Analyse (C₂₂H₃₂N₄S₂O₄ x 0,5 H₂O): | | | | |
|---|---|---|---|---|
| berechnet | 54,0% C, | 6,8% H, | 11,4% N, | 13,1% S; |
| gefunden | 54,1% C, | 6,8% H, | 11,3% N, | 12,8% S. |

### Beispiel 11

### 4-[N-[2-(4-Fluorphenyl)-2-hydroxyethyl]-N-methylamino]-1-(4-nitrophenyl)-piperidin

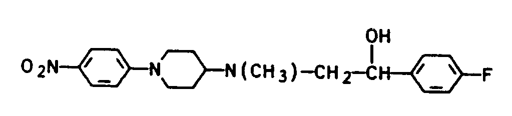

Eine Mischung aus 3,0 g (12,8 mmol) 4-(Methylamino)-1-(4-nitrophenyl)-piperidinfumarat, 1,8 g (12,8 mmol) 4-Fluorstyryloxid und 100 ml Ethanol wurde 3 Stunden auf Rückflußtemperatur erhitzt. Anschließend entfernte man das Lösungsmittel unter reduziertem Druck und verteilte den Rückstand zwischen Essigsäureethylester und Wasser. Die organische Phase wurde abgetrennt, getrocknet und unter reduziertem Druck eingeengt. Die Reinigung erfolgte mittels Chromatographie an Kieselgel (Laufmittel: Methylenchlorid/Methanol 10:1).
Ausbeute: 14 %; Fp.: 91-92°C

### Vorstufe 11.1

### 4-(Methylamino)-1-(4-nitrophenyl)-piperidin-fumarat

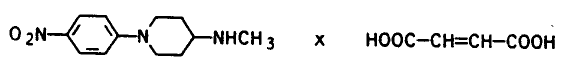

Zu einer mit Methylamin gesättigten Lösung aus 43 ml Essigsäure in 400 ml Methanol wurden portionsweise 15,0 g (68 mmol) 1-(4-Nitrophenyl)-4-piperidinon [vgl. E. Taylor et al., Synthesis, 606 (1981)] und danach 4,3 g (68 mmol) Natriumcyanoborhydrid gegeben. Nach 16 Stunden Rühren bei etwa 20°C entfernte man das Lösungsmittel unter reduziertem Druck und verteilte den Rückstand zwischen Methylenchlorid und Wasser. Die organische Phase wurde wie üblich auf das Reaktionsprodukt hin aufgearbeitet. Das Produkt wurde als Furmarat kristallisiert.
Ausbeute: 73 %; Fp.: 189-190°C

### Beispiel 12

### 4-[N-[2-Hydroxy-2-(4-nitrophenyl)eth-1-yl]-N-methylamino]-1-(4-nitrophenyl)-piperidin

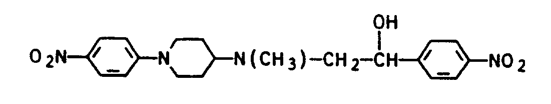

Eine Mischung aus 3,0 g (12,8 mmol) 4-(Methylamino)-1-(4-nitrophenyl)-piperidin, 2,6 g (25,5 mmol) Triethylamin, 3,1 g (12,8 mmol) 2-Brom-1-(4-nitrophenyl)-ethanol und 150 ml Methanol wurde 1 Stunde bei 20°C gerührt. Anschließend verteilte man die Reaktionsmischung zwischen gesättigter wäßriger Kaliumcarbonat-Lösung und Methylenchlorid. Anschließend wurde die organische Phase abgetrennt, getrocknet und unter reduziertem Druck eingeengt. Die Reinigung des Rohproduktes erfolgte mittels Chromatographie an Kieselgel (Laufmittel: Toluol/Aceton 1:1).
Ausbeute: 85 %.

| Analyse C₂₀H₂₄N₄O₅: | | | |
|---|---|---|---|
| berechnet | 60,0% C, | 6,0% H, | 14,0% N; |
| gefunden | 60,1% C, | 6,1% H, | 13,9% N. |

### Beispiel 13

### 4-[N-[2-Hydroxy-2-(4-methansulfonamido-phenyl)eth-1-yl]-N-methylamino]-1-(4-methansulfonylamido-phenyl)-piperidin

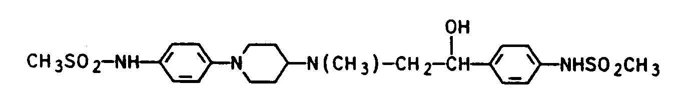

Eine Lösung von 4,0 g (10,0 mmol) 4-[N,N-[2-Hydroxy-2-(4-nitrophenyl)-1-ethyl]-methylamino]-1-(4-nitrophenyl)-piperidin in 200 ml Methanol wurde mit 0,5 g Palladium/Kohle (enthaltend 10 Gew.-% Palladium) versetzt. Man hydrierte bis zur Beendigung der Wasserstoffaufnahme, filtrierte dann die festen Bestandteile ab und entfernte das Lösungsmittel unter reduziertem Druck. Das entstandene Dianilinderivat wurde analog Beispiel 10 mit Methansulfonsäurechlorid umgesetzt.
Ausbeute: 0,45 g;
¹H-NMR (in D6-DMSO; TMS als interner Standard): 1,5 ppm (2H), 1,7 ppm (2H), 2,3 ppm (2H), 2,5 ppm (3H), 2,6 ppm (1H), 2,9 ppm (3H), 3,0 ppm (3H), 3,2 ppm (2H), 3,7 ppm (2H), 4,1 ppm (1H), 4,6 ppm (1H), 6,9 ppm (2H), 7,05 ppm (2H), 7,1 ppm (2H), 7,3 ppm (2H), 9,2 ppm (1H) und 9,7 ppm (1H).

### Beispiel 14

### 1-(4-Chlorphenyl)-4-[N-[2-(4-fluorphenyl)-2-hydroxyeth-1-yl]-N-methylamino)-piperidin-dioxalat

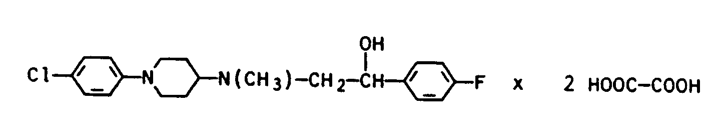

Eine Mischung aus 2,0 g (8,9 mmol) 1-(4-Chlorphenyl)-4-(N-methylamino)-piperidin, 1,2 g (8,9 mmol) 4-Fluorstyryloxid und 100 ml Ethanol wurde 5 Stunden auf Rückflußtemperatur erhitzt. Anschließend entfernte man das Lösungsmittel, wonach das Rohprodukt mittels Chromatographie an Kieselgel (Laufmittel Toluol/Aceton 1:1) gereinigt wurde. Das Produkt kristallisierte als Bisoxalat.
Ausbeute: 2,3 g; Fp.: 91-92°C

### Vorstufe 14.1

### 4-(N-Methyl-N-trifluoracetylamino)-1-(4-nitrophenyl)-piperidin

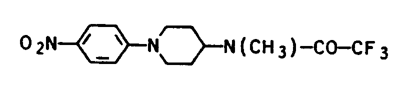

Zu einer Lösung aus 3,6 g (15,3 mmol) 4-(Methylamino)-1-(4-nitrophenyl)-piperidinfumarat (vgl. Vorstufe 11.1) in 50 ml wasserfreiem Tetrahydrofuran wurden 2,4 g (30,6 mmol) Pyridin gegeben. Anschließend versetzte man die Mischung bei 0°C tropfenweise mit einer Lösung aus 3,5 g (16,8 mmol) Trifluoressigsäureanhydrid in 10 ml wasserfreiem Tetrahydrofuran. Nach 72 Stunden Rühren bei 20 bis 25°C wurde das Lösungsmittel unter reduziertem Druck entfernt und der Rückstand wurde zwischen Wasser und Methylenchlorid verteilt. Danach wurde die organische Phase wie üblich auf das Produkt hin aufgearbeitet.
Ausbeute: 87 %; Fp.: 104-105°C

### Vorstufe 14.2

### 1-(4-Aminophenyl)-4-(N-methyl-N-trifluoracetylamino)-piperidin

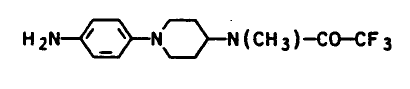

Eine Lösung von 3,8 g (11,5 mmol) 4-(N-Methyl-trifluoracetylamino)-1-(4-nitrophenyl)-piperidin in 100 ml Methanol wurde an einem Kontakt aus Palladium/Kohle (10 Gew.-% Palladium) hydriert. Nach beendeter Wasserstoff-Aufnahme wurden die festen Bestandteile aus der Mischung abgefiltert, wonach man das Lösungsmittel unter reduziertem Druck entfernte.
Ausbeute: 96 %; Fp.: 120-121°C

### Vorstufe 14.3

### 1-(4-Chlorphenyl)-4-(N-methylamino)-piperidin

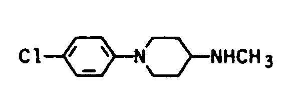

### Hilfslösung:

Eine Lösung von 2,7 g Kupfer-II-sulfat x 5 H₂O und 1,0 g Natriumchlorid in 8,8 ml Wasser wurde mit einer Lösung von 0,7 g Natriumsulfit in 2,2 ml Wasser versetzt. Entstandene feste Bestandteile wurden abgetrennt und in konzentrierter Salzsäure gelöst, wonach man beide Lösungen vereinigte.

Eine Lösung von 3,3 g (10,9 mmol) 1-(4-Aminophenyl)-4-(N-methyl-trifluoracetylamino)-piperidin in 3,7 ml 32 gew.-%iger Salzsäure wurde nacheinander mit 2 ml Wasser und, bei 0°C, tropfenweise mit einer Lösung von 0,76 g Natriumnitrit in 4,4 ml Wasser versetzt. Nach rascher Zugabe der Hilfslösung wurde die Mischung bis zur Beendigung der Gasentwicklung auf dem Wasserbad erhitzt, dann auf Eis gegossen und mit konzentrierter wäßriger Ammoniak-Lösung versetzt. Anschließend extrahierte man das Produkt mit Methylenchlorid und isolierte es danach wie üblich.
Ausbeute: 96 %; Fp.: 44-46°C

### Beispiel 15

### 1-(4-Chlorphenyl)-4-(4-phenyl-1-piperidinyl)-piperidin

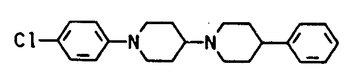

Analog Vorstufe 14.3 wurden 1,0 g (3 mmol) 1-(4-Aminophenyl)-4-(4-phenyl-1-piperidinyl)-piperidin (vgl. Bsp. 9) diazotiert und anschließend in das Chlorderivat überführt.
Ausbeute: 72 %; Fp.: 173°C

### B) Antiarrhythmische Wirkung

Die Wirkung der Phenylpiperidine I als Repolarisationshemmstoffe kann man durch EKG-Messungen belegen. Dabei unterteilt man die Herzperiode zeitlich in Systole (Zusammenziehen des Herzens), auch QT-Dauer genannt, und Diastole (Erschlaffung des Herzens mit Blutfüllung der Herzkammern). Repolarisationshemmstoffe bewirken nun eine Verlängerung der QT-Dauer ohne die Vorhofkammer-Überleitungszeit (PQ-Dauer) und die Anspannungszeit (QRS-Dauer, Beginn der Systole bis zum öffnen der Semilunarklappen) wesentlich zu verändern (s. Pschyrembel, 254. Auflage, 1982).

Die Wirksamkeit der erfindungsgemäßen Verbindungen als Repolarisationshemmstoffe läßt sich im Tierversuch durch EKG-Messungen beispielsweise am Meerschweinchenherzen untersuchen (s. Basic Res. Cardiol. 82 (1987) 437; J. Pharmacol. Methods 21 (1989) 195). Zum Vergleich der Wirksamkeit mehrerer Substanzen dient dabei zum Beispiel die Dosis eines Wirkstoffes, bei der eine 20 %ige Zunahme des QT-Ausgangswertes eintritt (ED_{20 %}). Hierzu trägt man die logarithmierten Dosiswerte der jeweiligen Substanzen gegen die experimentell gefundenen relativen Änderungen der QT-Dauer auf, und ermittelt mittels linearer Regression die Funktionsgleichung einer Geraden, aus der man dann den ED_{20 %}-Wert berechnen kann.

Mit dieser Methode wurden die ED_{20 %}-Werte erfindungsgemäßer Verbindungen bestimmt (s. Tabelle 1). Als Vergleichssubstanz diente D-Sotalol [4'-(1-Hydroxy-2-(isopropylamino)-ethyl)methansulfonanilid].

Als Versuchstiere dienten männliche Duncin-Hartley-Meerschweinchen mit einem Körpergewicht von 300 bis 350 g. 30 min nach Applikation von 1250 I.E. Heparin/kg Körpergewicht in den Bauchraum wurden die Tiere durch Genickschlag getötet. Nach dem Durchtrennen der Kopfschlagadern zum Entbluten, wurde der Bruststamm geöffnet, das Herz herausgetrennt und an eine Perfusionsapparatur angeschlossen. Die Perfusion erfolgte nach Langendorff mit Sauerstoff angereicherter auf 37°C erwärmter Krebs-Henseleit-Lösung (NaCl 6896 mg/l; KCl 350 mg/l; MgSO₄ 285 mg/l; CaCl₂ 370 mg/l; KH₂PO₄ 161 mg/l; NaHCO₃ 2090 mg/l; Glukose 2000 mg/l). Dabei wurde das Perfusionsvolumen pro Zeiteinheit bei einem Gesamtvolumen von 100 ml auf 4 bis 6 ml/min und der Perfusionsdruck auf 60 bis 70 mm Hg eingestellt. Nach einer Äquilibrierzeit von 30 min wurde zirkulierend perfundiert.

Die EKG-Messungen wurden über zwei Silberelektroden, die auf der Herzoberfläche im oberen Bereich der linken Koronararterie und auf der Rückseite des Herzens auf Höhe der Ventilebene angebracht waren, aufgenommen. Gemessen wurden die PQ-, QT- und QRS-Zeiten sowie die Herzfrequenz.

Die Substanzgabe erfolgte kumulativ im Abstand von 15 min ins Perfusat.

**Tabelle 1**

| Die QT-Zeit verlängernde Wirkung von substituierten N-Phenylpiperidinen I im Vergleich zu D-Sotalol. | |
|---|---|
| Beispiel Nr. | ED_{20 %} [»mol/l] |
| 2 | 0,42 |
| 3 | 0,031 |
| 10 | 0,96 |
| 11 | 0,21 |
| D-Sotalol | 16,0 |

### C) Bindung an Sigma-Rezeptor

Der verwendete Bindungsassay (Bindung von [³H]-Ditolylguanidin) erfaßt sogenannte "Haloperidolsensitive-Sigma-Rezeptoren", die eine hohe Affinität zum Haloperidol, aber nur geringe Affinität zum Phencyclidin und zu Opoiden aufweisen.

Als Referenzsubstanz wurde 1,3-Di-o-tolyl-guanidin (DTG), der selektive Ligand dieser Bindungsstelle [vgl. Life Sciences 47, 1073 (1990)], eingesetzt.

### Methoden:

### α) Membranpreparation

Rattengroßhirne wurden in dem 10fachen Volumen Homogenisierungspuffer (50 mmol Tris(hydroxymethyl)-amino-methan, 0,1 mmol Ethylendiamintetraacetat, pH=7,7) mit einem Polytron-Homogenisiator homogenisiert (20 sec.). Nach 15 Minuten Zentrifugation bei 40000 Umdrehungen/min wurde das erhaltene Pellet resuspendiert und die Suspension erneut 15 Minuten lang mit 40000 Umdrehungen/min zentrifugiert. Das hierbei erhaltene Pellet wurde im 5fachen Volumen Homogenisierungspuffer resuspendiert und bis zur weiteren Verwendung in flüssigem Stickstoff eingefroren.

### β) Sigma-Bindungstest

Testsubstanz und Membranen (0,3 mg Protein) wurden in 0,3 ml Inkubationspuffer (5 mmol Tris(hydroxymethyl)-amino-methan, 0,1 mmol Ethylendiamintetraacetat, pH=7,7) 45 Minuten bei 37°C inkubiert. Nach Zugabe von 100 000 dpm [³H]-Ditolylguanidin (54,5 Ci/mmol) wurde noch 1 Stunde inkubiert. Die Membranen wurden über GF/B-Filter (dunn-Labortechnik, Asbach) filtriert und mit einem 37°C warmen Waschpuffer (5 mmol Tris(hydroxymethyl)-amino-methan, 0,1 mmol Ethylendiamintetraacetat, pH=7,4) gewaschen. Die verbleibende Radioaktivität auf den Filtern wurde durch Flüssig-Scintillationszählung gemessen. Die Bindungsdaten wurden durch iterative Anpassungsprogramme analysiert.

Die Aktivitätkonstanten Kᵢ von substituierten N-Phenylpiperidinen I sind Tabelle 2 zu entnehmen.

**Tabelle 2**

| Aktivitätskonstanten Kᵢ für die [³H]-Ditolylguanidin-Bindungsstelle | |
|---|---|
| Beispiel Nr. | Kᵢ [nM] |
| 2 | 11 |
| 8 | 2,8 |
| DTG | 29,1 |

## Patentansprüche

1. Substituierte N-Phenylpiperidine der allgemeinen Formel I 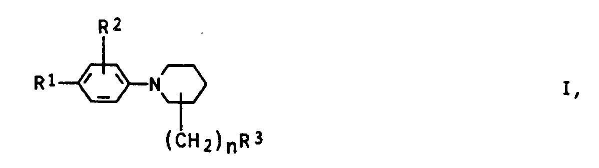 in der die Variablen die folgende Bedeutung haben:
R¹ Wasserstoff, Nitro, Cyano, Halogen, C₁-C₄-Alkyl, Trifluormethyl, Trifluormethoxy, Hydroxy, Hydroxymethyl, Hydroxycarbonyl, Formyl, Formylamino, Amino, Aminocarbonyl, Tetrazin-5-yl, R⁴-O-, R⁴-O-CH₂-, R⁴-O-CO-, R⁴-CO-, R⁴-NH-CO, R⁴-CO-NH-, R⁴-SO₂-NH-;
R² Wasserstoff, Nitro, Halogen, C₁-C₄-Alkyl oder R⁴-O-;
R³ einen der folgenden Reste: 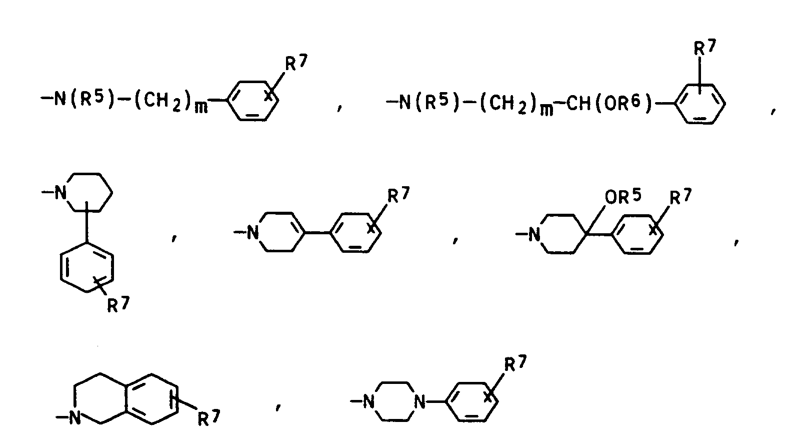
R⁴ C₁-C₄-Alkyl oder Phenyl, das noch einen der Reste R² tragen kann;
R⁵,R⁶ Wasserstoff, C₁-C₄-Alkyl oder Phenyl, das noch einen der Reste R² tragen kann;
R⁷ einer der Reste R¹;
n 0 oder 1;
m 1 oder 2;
mit der Maßgabe, daß R³ nur dann 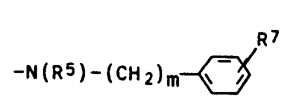 bedeutet, wenn n 1 ist,
im Falle optischer Isomerie auch die optischen Isomeren, sowie die physiologisch verträglichen Säureadditionssalze.

2. Substituierte N-Phenylpiperidine der allgemeinen Formel I gemäß Anspruch 1 zur Verwendung auf dem Arzneimittelgebiet.

3. Arzneimittel, enthaltend übliche Hilfsstoffe und eine therapeutisch wirksame Menge eines N-Phenylpiperidins der Formel I gemäß Anspruch 1.

4. Verwendung der Verbindungen I und deren Salzen mit physiologisch verträglichen Säuren gemäß Anspruch 1 zur Herstellung von Arzneimitteln.

## Claims

1. A substituted N-phenylpiperidine of the formula I 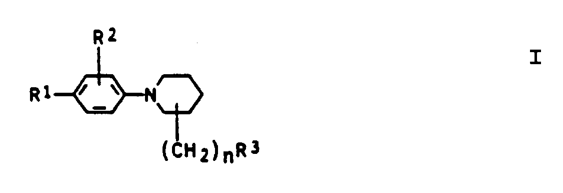 where
R¹ is hydrogen, nitro, cyano, halogen, C₁-C₄-alkyl, trifluoromethyl, trifluoromethoxy, hydroxyl, hydroxymethyl, hydroxycarbonyl, formyl, formylamino, amino, aminocarbonyl, 5-tetrazinyl, R⁴-O-, R⁴-O-CH₂-, R⁴-0-CO-, R⁴-CO-, R⁴-NH-CO, R⁴-CO-NH-, R⁴-SO₂-NH-;
R² is hydrogen, nitro, halogen, C₁-C₄-alkyl or R⁴-O-;
R³ is one of the following: 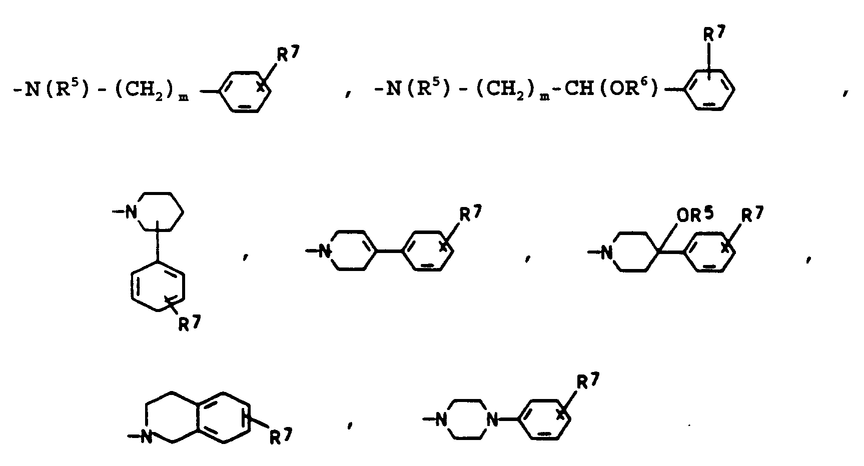
R⁴ is C₁-C₄-alkyl or phenyl which may carry one of the R² radicals;
R⁵ and R⁶ are each hydrogen, C₁-C₄-alkyl or phenyl which may carry one of the R² radicals;
R⁷ is one of the R¹ radicals;
n is 0 or 1;
m is 1 or 2;
with the proviso that R³ can be 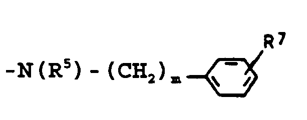 only when n is 1,
and the optical isomers when there is optical isomerism, and the physiologically tolerated acid addition salts.

2. A substituted N-phenylpiperidine of the formula I as claimed in claim 1 for use in pharmaceuticals.

3. A drug containing conventional auxiliaries and a therapeutically effective amount of an N-phenylpiperidine of the formula I as claimed in claim 1.

4. The use of a compound I or a salt thereof with a physiologically tolerated acid as claimed in claim 1 for the production of drugs.

## Revendications

1. N-phénylpipéridines substitués de formule générale I 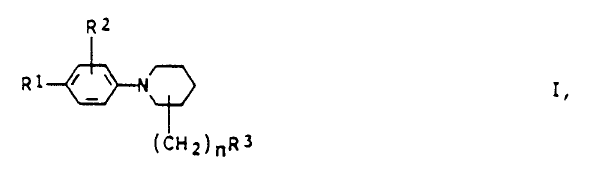 dans laquelle les variables ont la signification suivante :
R¹ hydrogène, nitro, cyano, halogène, alkyle en C1-C4, trifluorométhyle, trifluorométhoxy, hydroxy, hydroxyméthyle, hydroxycarbonyle, formyle, formylamino, amino, aminocarbonyle, tétrazin-5-yle, R⁴-O-, R⁴-O-CH₂-, R⁴-O-CO-, R⁴-CO-, R⁴-NH-CO, R⁴-CO-NH-, R⁴-SO₂-NH-;
R² hydrogène, nitro, halogène, alkyle en C1-C4 ou R⁴-O-;
R³ un des restes suivants : 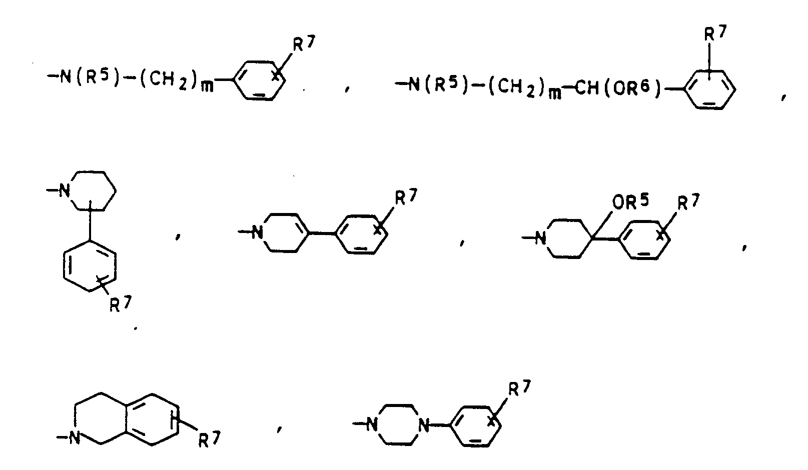
R⁴ alkyle en C1-C4 ou phényle qui peut encore porter un des restes R²;
R⁵, R⁶ hydrogène, alkyle en C1-C4 ou phényle, qui peut encore porter un des restes R²;
R⁷ un des restes R¹
n 0 ou 1 ;
m 1 ou 2 ;
sous réserve que R³, lorsque n = 1, ne signifie alors que 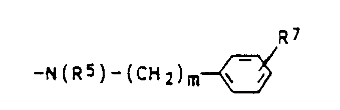 dans le cas d'isomère optique, aussi les isomères optiques ainsi que les sels d'addition d'acides acceptables physiologiquement.

2. N-phénylpipéridines substitués de formule générale I selon la revendication 1 pour utilisation dans le domaine des médicaments.

3. Médicaments contenant des auxiliaires usuels et une quantité efficace en thérapeutie d'une N-phénylpipéridine de formule I selon la revendication 1.

4. Utilisation des composés I et leurs sels avec des acides acceptables physiologiquement selon la revendication 1 pour la préparation de médicaments.
